# EUROPÄISCHE PATENTSCHRIFT

(11) **EP 0 384 983 B1**
(45) Veröffentlichungstag und Bekanntmachung des Hinweises auf die Patenterteilung: **09.06.1993**
(21) Anmeldenummer: 89123899.0
(22) Anmeldetag: 23.12.1989
(51) Int. Cl.: A61K 7/50, A61K 7/08, C11D 1/83

(54) **Tensidkombination**
Detergent composition
Composition détergente

(30) Priorität: 25.02.1989 DE 3905939
(43) Veröffentlichungstag der Anmeldung: 05.09.1990
(73) Patentinhaber: HÜLS AKTIENGESELLSCHAFT, 45764 Marl (DE)
(72) Erfinder: Balzer, Dieter, Dr., D-4358 Haltern (DE)

(56) Entgegenhaltungen:
- EP-A- 0 070 075
- EP-A- 0 176 151
- US-A- 4 240 921

## Beschreibung

Die Erfindung betrifft Tensidkombinationen als Basis für Shampoonierungsmittel, Bade- und Duschgele sowie andere kosmetische Formulierungen auf wäßriger Basis bzw. flüssige Reinigungsmittel oder andere Systeme, für die aus Applikationsgründen eine erhöhte Viskosität notwendig ist.

In der Vergangenheit basierten solche Systeme vorwiegend auf Alkylsulfaten oder Alkylethersulfaten mit gewöhnlich niedrigem Ethoxylierungsgrad. Die Viskosität hierbei läßt sich relativ einfach durch Zugabe von Kochsalz, Ammoniumchlorid eventuell kombiniert mit Fettsäurediethanolamid und/oder anderen Additiven einstellen. Solche Formulierungen zeigen zwar befriedigenden Schaum und sind preiswert, haben allerdings den großen Nachteil starker Hautreizung sowie Augenschleimhautreizung, was in Anbetracht der immer häufiger werdenden, zum Teil täglichen Anwendung von erheblichem Gewicht ist. Aufgrund des Risikopotentials von Spurenverunreinigungen an N-Nitrosodiethanolamin ist es darüber hinaus sinnvoll, möglichst N-freie Formulierungen zur Verfügung zu haben (vgl. Hamke Meijer, Seifen-Öle-Fette-Wachse 114, 159 (1988).

Die Suche nach milderen Tensidpräparationen, die nicht die o. a. Nachteile beinhalten, sich aber gleichzeitig einfach verdicken lassen, ist somit angezeigt. Es taucht hierbei das Problem auf, daß haut- bzw. schleimhautfreundliche, preiswerte Tenside keine genügende Elektrolytverdickbarkeit (H. Meijer, Seifen-Öle-Fette-Wachse 113, 135 (1987) und H. Tesmann, Parfümerie und Kosmetik 68, 630 (1987) aufweisen. Ausreichend hohe Viskosität bzw. Verdickbarkeit versucht man daher entweder durch Erhöhung der Tensidkonzentration oder mittels einer nur begrenzten Teilsubstitution des Ethersulfats durch ein milderes und toxikologisch unbedenklicheres Tensid zu erzielen (H. Meijer, loc. cit und US-PS 3 038 862), was den eben aufgeführten Nachteilen aber nicht gerecht wird. Eine Verdickung mittels wasserlöslicher Polymere gilt hierbei wegen der Beeinflussung der Schaumqualität sowie des Hautgefühls als ungeeignete Alternative.

Als milde haut- bzw. schleimhautverträgliche Tenside gelten die carboxymethylierten Oxethylate, die in dieser Hinsicht als sehr gut zu bezeichnen sind (N.A.I., Seifen-Öle-Fette-Wachse 109, 353 (1983). Aber diese Oxethylate besitzen den entscheidenden Nachteil, daß sie mit üblichen Elektrolytkonzentrationen nicht oder nur unzureichend verdickbar sind (EP-A 0 176 151).

Aber auch die Alkyloligoglycoside weisen sehr gute Werte hinsichtlich der Schleimhautverträglichkeit auf (A.D. Urfer et al., Poster-Vortrag, Second World Conference on Detergents, Montreux, 1986). Aber hier zeigt sich für die Alkyloligoglycoside, insbesondere mit einem Glycosidierungsgrad von 1 bis 1,45, eine völlig unzureichende Elektrolytverdickbarkeit. Solche Alkyloligoglycoside sind bei Konzentrationen unter 10 Gew.-% gewöhnlich in Wasser unlöslich oder zumindest schwerlöslich, was in Gegenwart von Elektrolyt zu stark trüben oder sogar zu 2-phasigen Dispersionen führt.

Es bestand daher die Aufgabe, elektrolytverdickbare, haut- und augenschleimhautfreundliche Tensidkombinationen aufzufinden, die weitgehend frei von Ethersulfaten und N-haltigen Tensiden sind.

Diese Aufgabe wurde dadurch gelöst, daß man Mischungen aus aus 3 - 30 Gew.-% carboxymethyliertem Alkenoloxethylat allein oder einer Kombination aus carboxymethyliertem Alkenoloxethylat mit carboxymethyliertem Alkanoloxethylat oder carboxymethyliertem Alkylphenoloxethylat mit 7 bis 18 C-Atomen in der geradkettigen oder verzweigten Alkylgruppe allein oder einer Kombination aus carboxymethyliertem Alkylphenoloxethylat mit 7 bis 18 C-Atomen in der geradkettigen oder verzweigten Alkylgruppe mit carboxymethyliertem Alkenoloxethylat,
3 - 30 Gew.-% Alkyloligoglycosid, 0,05 - 5 Gew.-% elektrolytischem Verdickungsmittel, Wasser und gegebenenfalls Additiven bestehend, herstellt.

Gegenstand der Erfindung ist daher eine Tensidkombination für Badegele, Shampoos und Reinigungsmittel bestehend aus
a) 3 - 30 Gew.-% carboxymethyliertem Alkenoloxethylat allein
   oder einer Kombination aus carboxymethyliertem Alkenoloxethylat mit carboxymethyliertem Alkanoloxethylat
   oder carboxymethyliertem Alkylphenoloxethylat mit 7 bis 18 C-Atomen in der geradkettigen oder verzweigten Alkylgruppe allein
   oder einer Kombination aus carboxymethyliertem Alkylphenoloxethylat mit 7 bis 18 C-Atomen in der geradkettigen oder verzweigten Alkylgruppe mit carboxymethyliertem Alkenoloxethylat,
b) 3 - 30 Gew.-% Alkyloligoglycosid,
c) 0,05 - 5 Gew.-% elektrolytischem Verdickungsmittel,
d) gegebenenfalls Additiven und
   Wasser ad 100 Gew.-%.

Die Verwendung von Kombinationen der Alkylpolyglycoside oder Alkyloligoglycoside mit anionischen Tensiden ist seit langem bekannt.

So beschreibt bereits die DRP 593 422 die Verwendung von Cetylmaltosid mit gewöhnlicher Seife, US-PS 3 721 633 betrifft die Verwendung von Gemischen aus Alkylpolyglycosiden mit anionischen synthetischen Tensiden, wie z. B. Dodecylbenzolsulfonat, und das Technical Bulletin Triton CG 100 der Rohm und Haas von 1975 erwähnt eine Abmischung des Alkylpolyglycosids mit u. a. Laurylethersulfat.

WO 86/02943 beschreibt die Kombination von Alkylmonoglycosiden bzw. Alkyloligoglycosiden mit unterschiedlichen Aniontensiden wie Alkylsulfat, Alkylethersulfat, Olefinsulfonat, Paraffinsulfonat oder Alkylbenzolsulfonat mit dem Ziel einer Viskositätserhöhung. Im Unterschied zur vorliegenden Erfindung sind die beanspruchten Aniontenside wenig haut- und augenschleimhautverträglich, was von erheblichem Gewicht ist.

Die Kombination von Alkylpolyglycosiden mit Alkylbenzolsulfonat und unter anderem carboxymethylierten Alkanoloxethylaten wird in der EP 0 070 075 erwähnt, wobei ein deutlich höherer Glycosidierungsgrad der Glycoside vorliegt. Die Elektrolytverdickbarkeit, wie sie die erfindungsgemäßen Kombinationen aus Alkyloligoglycosiden und carboxymethylierten Alkenoloxethylaten, gegebenenfalls kombiniert mit carboxymethylierten Alkanoloxethylaten, aufweisen, ist nicht gegeben.

Es wurde nun überraschend gefunden, daß sich die erfindungsgemäße Kombination aus Alkyloligoglycosiden und carboxymethylierten Alkenoloxethylaten, gegebenenfalls kombiniert mit carboxymethylierten Alkanoloxethylaten und die erfindungsgemäße Kombination aus Alkyloligoglycosiden und carboxymethylierten Alkylphenoloxethylaten mit 7 bis 18 C-Atomen in der geradkettigen oder verzweigten Alkylgruppe, gegebenen falls Kombiniert mit carboxymethylierten Alkenoloxyethylaten, im Gegensatz zu ihren Einzelkomponenten mittels üblicher Elektrolytkonzentrationen außerordentlich gut verdicken läßt. Es liegt ein starker synergistischer Verdickungseffekt vor; die Lösungen bleiben nach der Elektrolytzugabe klar und zeigen selbst bei Konzentrationen an waschaktiver Substanz von nur 10 Gew.-% zum Teil gelartiges Fließverhalten.

### Carboxymethylierte Oxethylate:

Erfindungsgemäß eingesetzte carboxymethylierte Oxethylate entsprechen der Formel (I)

R-(OC₃H₆)ₘ(OC₂H₄)ₙOCH₂COOM, (I)

in der R einen linearen oder verzweigten, gesättigten oder ungesättigten Kohlenwasserstoffrest mit 10 bis 20 Kohlenstoffatomen, einen alkylaromatischen Rest mit 7 bis 18 C-Atomen in der geradkettigen oder verzweigten, gesättigten oder ungesättigten Alkylgruppe bzw. Gemische davon, m 0 bis 10, n 1 bis 15, M ein Alkali-, Erdalkalimetall-, Ammonium- oder Alkylammoniumion bedeuten, wobei die Oxethylate auf Basis gesättigter Fettalkohole nur in Verbindung mit Oxethylaten auf der Basis ungesättigter Alkohole eingesetzt werden. Bevorzugt werden carboxymethylierte Oxethylate auf der Basis hydrophober Fettalkohole mit 14 bis 20 C-Atomen und m = 0. Besonders bevorzugt werden carboxymethylierte Oxethylate auf der Basis ungesättigter Fettalkohole oder Gemische von gesättigten und ungesättigten Fettalkoholen.

Die carboxymethylierten Oxethylate können z. B. nach der DE-PS 2 418 444 durch Umsetzung von Oxethylaten der Formel R-(OC₂H₄)ₙH oder R-(OC₃H₆)ₘ(OC₂H₄)ₙH mit einem Salz der Chloressigsäure in Gegenwart von Alkalihydroxid oder anderen Basen hergestellt werden. Die Umsetzung muß hierbei nicht quantitativ sein, so daß das carboxymethylierte Oxethylat ein Gemisch von Ausgangsoxethylat und Umsetzungsprodukt ist.

Abhängig von der Verwendung kann das mitentstehende Salz in vielen Fällen in dem Produkt verbleiben. Geeignet insbesondere für kosmetische Formulierungen sind carboxymethylierte Oxethylate auf der Basis Oleylalkohol, Elaidylalkohol, Linoleylalkohol, Linolenylalkohol etc. sowie deren Gemische auch mit langkettigen Alkanolen.

### Alkyloligoglycoside:

Erfindungsgemäß eingesetzte Glycoside sind Alkyoligoglycoside, das heißt Verbindungen der Formel (II)

R′-O-Zₙ (II)

in der R′ für einen linearen oder verzweigten gesättigten oder ungesättigen aliphatischen Alkylrest mit 8 bis 16 Kohlenstoffatomen oder Gemische davon und Zₙ für einen Oligoglycosylrest mit n = 1 bis 1,45, bevorzugt 1,1 bis 1,45, Hexose- oder Pentoseeinheiten oder Gemische davon steht. Besonders bevorzugt werden Alkyloligoglycoside mit 9 bis 14 C-Atomen.

Die erfindungsgemäß eingesetzten Alkyloligoglycoside können nach bekannten Verfahren ganz oder teilweise auf Basis nachwachsender Rohstoffe hergestellt werden. Beispielsweise wird Dextrose in Gegenwart eines sauren Katalysators mit n-Butanol zu Butyloligoglycosidgemischen umgesetzt, welche mit langkettigen Alkoholen ebenfalls in Gegenwart eines sauren Katalysators zu den gewünschten Alkyloligoglycosidgemischen umglycosidiert werden. Die Formel der Produkte ist in bestimmten Grenzen variierbar. Der Alkylrest R′ wird durch die Auswahl des langkettigen Alkohols festgelegt. Günstig aus wirtschaftlichen Gründen sind die großtechnisch zugänglichen Tensidalkohole mit 8 bis 16 C-Atomen, z. B. Oxoalkohole, Ziegleralkohole und native Alkohole aus der Hydrierung von Fettsäuren bzw. Fettsäurederivaten.

Der Oligoglycosylrest Zₙ wird einerseits durch die Auswahl des Kohlenhydrats und andererseits durch die Einstellung des mittleren Oligomerisationsgrades n z. B. nach DE-OS 19 43 689 festgelegt. Im Prinzip können bekanntlich Polysaccharide, Oligosaccharide und Monosaccharide, z. B. Stärke, Maltodextrine, Dextrose, Galaktose, Mannose, Xylose usw. zu Alkyloligoglycosiden umgesetzt werden. Besonders bevorzugt sind die großtechnisch verfügbaren Kohlenhydrate Stärke, Maltodextrine und Dextrose. Da die wirtschaftlich interessanten Alkyloligoglycosidsynthesen nicht regio- und stereoselektiv verlaufen, sind die Alkyloligoglycoside stets Gemische von Oligomeren, die ihrerseits Gemische verschiedener isomerer Formen darstellen. Sie liegen nebeneinander mit α- und β-glycosidischen Bindungen in Pyranose- und Furanoseform vor. Auch die Verknüpfungsstellen zwischen zwei Saccharidresten sind unterschiedlich.

Erfindungsgemäß eingesetzte Alkyloligoglycoside lassen sich auch durch Abmischen von Alkylpolyglycosiden mit Alkylmonoglycosiden herstellen. Letztere kann man z. B. nach EP-A 0 092 355 mittels polarer Lösemittel wie Aceton aus Alkylpolyglycosiden gewinnen bzw. anreichern.

Die Mengenverhältnisse in der erfindungsgemäßen Kombination aus Alkyloligoglycosid und carboxymethyliertem Oxethylat liegen zwischen 5 : 1 und 1 : 10, bevorzugt werden Verhältnisse zwischen 2 : 1 und 1 : 5. Die Konzentrationen in wäßrigen Zübereitungen liegen zwischen 5 und 30 Gew.-% Aktivsubstanz, vorzugsweise zwischen 7 und 20 Gew.-%.

### Verdickungsmittel:

Als erfindungsgemäß eingesetzte Verdickungsmittel eignen sich Elektrolyte. Diese elektrolytischen Verdickungsmittel sind zum Beispiel Natriumchlorid, Ammoniumchlorid, Natriumsulfat, Magnesiumsulfat etc.

Die erfindungsgemäßen Kombinationen aus Alkyloligoglycosid und carboxymethyliertem Oxethylat sind in Abhängigkeit von ihrer Anwendung gewöhnlich ausreichend schäumend. Sind höhere Schaumnoten erwünscht, so empfiehlt sich zum Teil die Zugabe kleiner Mengen (höchstens 5 Gew.-%) stark schäumender Additive. Hierzu können stark schäumende anionische Tenside, wie z. B. organische Sulfate oder Sulfonate sowie Sorbitanester verwendet werden.

Die Mengenverhältnisse dieser Hilfstenside zu der erfindungsgemäßen Kombination betragen 2 : 8, vorzugsweise 1 : 9. In manchen aber im Hinblick auf ihre Umweltproblematik seltenen Fällen kann es nützlich sein, Fettsäureamide in kleinen Mengen mitzuverwenden.

Als weitere Additive in Abhängigkeit von den Anwendungen kommen kleine Mengen Polymere wie Polyethylenoxid, Chelatbildner, Konservierungsmittel, Duftstoffe etc. in Frage.

Mit den folgenden Beispielen soll die wirksame Verdickbarkeit der erfindungsgemäßen Kombination aufgezeigt werden. Sie wird nachgewiesen durch Viskositätsmessungen in einem Rotationsviskosimeter (Haake RV 20) bei 25 °C unter definierten Scherraten. Bei stark strukturviskosen Zubereitungen werden die mittleren Viskositäten bei Scherraten zwischen 3 und 10 sec⁻¹ mitgeteilt, bei Bedingungen also, die in etwa dem Bewegungsvorgang beim Ausfluß einer Flüssigkeit aus einer Plastikflasche mit mittlerer Öffnung entsprechen. Der Carboxymethylierungsgrad der carboxymethylierten Oxethylate liegt bei etwa 90 %.

### Beispiel 1 (erfindungsgemäß)

In einem Glasgefäß werden 5,5 g carboxymethyliertes Ocenol-80/85-Oxethylat-Na-Salz* mit 4 mol Ethylenoxid/mol, 9,26 g C₁₂C₁₃-Alkyloligoglycosid mit einem D.P. von 1,4 (bestimmt via ¹H-NMR) sowie 85,24 g dest. Wasser verrührt. Im carboxymethylierten Oxethylat sind 0,5 g NaCl, im Alkyloligoglycosid 4,26 g Wasser enthalten, so daß der Gehalt an waschaktiver Substanz 10 Gew.-% beträgt. Das Gemisch wurde erwärmt, es ergibt eine auch nach mehreren Tagen klare Lösung. Gleiches gilt auch bei höheren Kochsalzgehalten bis zu 3 Gew.-%. Die Viskosität in Abhängigkeit des Kochsalzgehaltes ist in der folgenden Tabelle zusammengefaßt:
*Ocenol 80.85: Oleyl-Cetylalkoholgemisch der Fa. Henkel

| NaCl (Gew.-%) | 0,5 | 1 | 1,5 | 2,0 | 3,0 |
|---|---|---|---|---|---|
| η 25 °C (mPa·s) | 2 | 10 | 115 | 580 | 2 800 |

### Beispiel 2 (Vergleichsbeispiel)

Mit diesem Beispiel soll gezeigt werden, daß eine 10%ige Lösung von carboxymethyliertem Ocenol-80/85-oxethylat mit 4 mol Ethylenoxid/mol (11,0 g pro 100 ml) durch NaCl-Zusatz nicht oder nur geringfügig verdickt wird (nachstehende Tab.). Auch diese Lösung ist klar.

| NaCl (Gew.-%) | 0,5 | 1 | 1,5 | 2,0 |
|---|---|---|---|---|
| η 25 °C (mPa·s) | 1 | 4 | 6 | 31 |

### Beispiel 3 (Vergleichsbeispiel)

Dieses Beispiel zeigt, daß eine 10%ige Lösung eines C₁₂C₁₃-Alkyloligoglycosids mit einem Glycosidierungsgrad von 1,4 (= 18,52 g/100 ml) durch Elektrolyt nicht verdickbar ist.

| NaCl (Gew.-%) | 0 | 1 | 2 | 3 |
|---|---|---|---|---|
| η 25 °C (mPa·s) | 10 | 2 | 5 | 6 |

### Beispiel 4 (erfindungsgemäß)

5,5 g carboxymethyliertes Ocenol-80/85-Oxethylat mit 3 mol Ethylenoxid/mol (enthält 10 Gew.-% NaCl) und 10,6 g C₁₀C₁₂-Alkyloligoglycosid mit einem Glycosidierungsgrad von 1,3 (Aktivgehalt 47 Gew.-%) werden in 83,9 g Wasser in der Wärme gelöst. Anschließend wird NaCl zugefügt.

Die Viskositätsmessung der klaren Lösung mit 10 Gew.-% waschaktiver Substanz in Abhängigkeit des NaCl-Gehaltes ergibt folgende Ergebnisse:

| NaCl (Gew.-%) | 0,5 | 1 | 1,5 | 2 | 2,5 |
|---|---|---|---|---|---|
| η 25 °C (mPa·s) | 25 | 360 | 660 | 2 730 | 1 900 |

### Beispiel 5 (erfindungsgemäß)

5,5 g carboxymethyliertes Ocenol-80/85-Oxethylat mit 4 mol Ethylenoxid/mol und 10,4 g C₁₂C₁₄-Alkyloligoglycosid mit einem Glycosidierungsgrad von 1,3 wurden in 84,1 g Wasser gelöst und sodann NaCl hinzugegeben bis die klare, etwas gelartige Lösung 1,5 Gew.-% NaCl enthielt. Die Konzentration an waschaktiver Substanz betrug 10 Gew.-%. Die Viskosität bei Scherraten zwischen 3 und 10 sec⁻¹ betrug 3 500 mPa·s.

### Beispiel 6 (erfindungsgemäß)

5,6 g carboxymethyliertes Ocenol-110/130*-Oxethylat mit 5 mol Ethylenoxid/mol, 10,4 g C₁₂C₁₄-Alkyloligoglycosid mit einem Glycosidierungsgrad von 1,3 wurden in 84 g Wasser gelöst und sodann mit NaCl versetzt. Der Gehalt an waschaktiver Substanz beträgt 10 Gew.-%. Die Viskositätsmessung in Abhängigkeit der NaCl-Konzentration des Systems ergab:
*Ocenol 110.130 ist ein Oleyl-Linoleyl-Alkohol der Fa. Henkel

| NaCl (Gew.-%) | 0,5 | 1,0 | 2,0 | 3,0 | 4,0 |
|---|---|---|---|---|---|
| η 25 °C (mPa·s) | 7 | 30 | 440 | 2 600 | 4 500 |

### Beispiel 7 (erfindungsgemäß)

5,4 g carboxymethyliertes Dodecylphenol-Oxethylat mit 6 mol Ethylenoxid/mol, 10,4 g C₁₂C₁₄-Alkyloligoglycosid (D.P. 1,3) und 1,4 g NaCl wurden in 83 g Wasser gelöst. Die Viskosität der ca. 10 Gew.-% waschaktiver Substanz enthaltenden Lösung lag bei 1 400 mPa·s.

### Beispiel 8 (erfindungsgemäß)

4,2 g carboxymethyliertes Ocenol-80/85-Oxethylat mit 5 mol Ethylenoxid/mol, 1,4 g carboxymethyliertes C₁₆C₁₈-Talgfettalkoholoxethylat mit 5 mol Ethylenoxid/mol und 10,4 g C₁₂C₁₄-Oligoglycosid (D.P. 1,3) werden in 84 g Wasser gelöst und sodann NaCl hinzugefügt. Die anschließende Viskositätsmessung der klaren, 10 Gew.-% waschaktive Substanz enthaltenden Lösung ergab die erfindungsgemäße Verdickbarkeit mittels zugesetztem Elektrolyt.

| NaCl (Gew.-%) | 1 | 2 | 3 |
|---|---|---|---|
| η 25 °C (mPa·s) | 31 | 215 | 2 950 |

### Beispiel 9 (erfindungsgemäß)

2,8 g carboxymethyliertes Ocenol-80/85-Oxethylat mit 4 mol Ethylenoxid/mol, 2,8 g carboxymethyliertes C₁₆C₁₈-Talgfettalkoholoxethylat mit 4 mol Ethylenoxid/mol und 10,4 g C₁₂C₁₄-Oligoglycosid (D.P. 1,3) werden in 84 g Wasser gelöst und sodann NaCl hinzugefügt. Die Viskosität der gelartigen Lösung (10 Gew.-% waschaktive Substanz, 3 Gew.-% NaCl) beträgt 4 800 mPa·s.

### Beispiel 10 (erfindungsgemäß)

8,2 g carboxymethyliertes Ocenol-92/96*-Oxethylat-Na-Salz mit 4 mol Ethylenoxid/mol, 5,2g C₁₂C₁₄-Alkyloligoglycosid (D.P. 1,25) wurden in 86,6 g Wasser gelöst. Sodann wurde NH₄Cl bis zu einer Konzentration von 2 Gew.-% hinzugefügt. Vom carboxymethylierten Oxethylat herstammend sind weitere 0,6 Gew.-% NaCl in dem Gemisch enthalten. Die Viskosität der 10 Gew.-% waschaktive Substanz enthaltenden, klaren Abmischung liegt bei 11 000 mPa·s.
*Oleylalkohol der Fa. Henkel

### Beispiel 11 (erfindungsgemäß)

5,4 g carboxymethyliertes Nonylphenoloxethylat, das zuerst mit 3 mol/mol Propylenoxid und sodann mit 6,1 mol/mol Ethylenoxid umgesetzt worden war, 10,4 g C₁₂C₁₄-Alkyloligoglycosid (D.P. 1,3) und 2 g NaCl wurden in 86,5 g Wasser gelöst. Die klare Lösung enthält 10 Gew.-% waschaktive Substanz und weist eine Viskosität von 750 mPa·s auf.

### Beispiel 12 (erfindungsgemäß)

4,3 g carboxymethyliertes Ocenol-80/85-Oxethylat mit 4 mol Ethylenoxid/mol, 8,5 g C₁₂C₁₄-Alkyloligoglycosid (D.P. 1,3), und 2,8 g MARLINAT 242 C₁₂₋₁₄-Alkanolethersulfat mit 2 mol Ethylenoxid/mol und 2 g NaCl wurden in 82,4 g Wasser gelöst. Die Viskositätsmessung der Lösung ergab 2 750 mPa·s, das Schäumvermögen - gemessen in einer manuellen Lochplatten-Schäumapparatur von 250 ml mit 1 g/l waschaktiver Substanz in Wasser von 12 °dh - ergab 285 ml Schäume, gemessen 30 sec nach Beendigung des Schlagens. Ein marktübliches Shampoo (Rilanet-Kurshampoo) ergab bei gleicher Konzentration an waschaktiver Substanz 260 ml Schaum.

## Patentansprüche

1. Tensidkombination für Badegele, Shampoos und Reinigungsmittel bestehend aus
a) 3 - 30 Gew.-% carboxymethyliertem Alkenoloxethylat allein
oder einer Kombination aus carboxymethyliertem Alkenoloxethylat mit carboxymethyliertem Alkanoloxethylat
oder carboxymethyliertem Alkylphenoloxethylat mit 7 bis 18 C-Atomen in der geradkettigen oder verzweigten Alkylgruppe allein
oder einer Kombination aus carboxymethyliertem Alkylphenoloxethylat mit 7 bis 18 C-Atomen in der geradkettigen oder verzweigten Alkylgruppe mit carboxymethyliertem Alkenoloxethylat,
b) 3 - 30 Gew.-% Alkyloligoglycosid,
c) 0,05 - 5 Gew.-% elektrolytischem Verdickungsmittel,
d) gegebenenfalls Additiven und
Wasser ad 100 Gew.-%.

2. Tensidkombination nach Anspruch 1,
dadurch gekennzeichnet,
daß als carboxymethylierte Oxethylate Verbindungen der Formel (I)
R-(OC₃H₆)ₘ(OC₂H₄)ₙOCH₂COOM (I),
in der R einen linearen oder verzweigten, ungesättigten aliphatischen Rest mit 10 bis 20 Kohlenstoffatomen, m 0 bis 10, n 1 bis 15, M ein Alkali-, Erdalkalimetall-, Ammonium- oder Alkylammoniumion bedeuten, eingesetzt werden, wobei der Carboxymethylierungsgrad zwischen 20 und 100 Gew.-% liegt.

3. Tensidkombination nach Anspruch 2,
dadurch gekennzeichnet,
daß in der Formel (I) R einen einfach- oder mehrfach ungesättigten Kohlenwasserstoffrest bedeutet.

4. Tensidkombination nach Anspruch 1,
dadurch gekennzeichnet,
daß als carboxymethylierte Oxethylate Verbindungen der Formel (I)
R-(OC₃H₆)ₘ(OC₂H₄)ₙOCH₂COOM (I),
in der R einen linearen oder verzweigten, gesättigten aliphatischen Rest mit 10 bis 20 Kohlenstoffatomen oder einem alkylaromatischen Rest mit 7 bis 18 Kohlenstoffatomen in der geradkettigen oder verzweigten, gesättigten oder ungesättigten Alkylgruppe und einen linearen oder verzweigten ungesättigten aliphatischen Rest mit 10 bis 20 Kohlenstoffatomen, m 0 bis 10, n 1 bis 15, M ein Alkali-, Erdalkalimetall-, Ammonium- oder Alkylammoniumion bedeuten, eingesetzt werden, wobei der Carboxymethylierungsgrad zwischen 20 und 100 Gew.-% liegt.

5. Tensidkombination nach Anspruch 1,
dadurch gekennzeichnet,
daß als Alkyloligoglycoside Verbindungen der Formel (II)
R'-O-Zₙ (II)
eingesetzt werden, wobei R' ein gesättigter oder ungesättigter, verzweigter oder unverzweigter Alkylrest mit 8 bis 16 Kohlenstoffatomen, Zₙ ein Oligoglycosylradikal bestehend aus 1 bis 1,45 Hexose- oder Pentoseeinheiten bzw. Mischungen davon bedeuten.

6. Tensidkombination nach Anspruch 5,
dadurch gekennzeichnet,
daß als Alkyloligoglycoside Verbindungen der Formel (II) eingesetzt werden, wobei R' ein gesättigter oder ungesättigter, verzweigter oder unverzweigter Alkylrest mit 9 bis 14 Kohlenstoffatomen bedeutet.

7. Tensidkombination nach Anspruch 5,
dadurch gekennzeichnet,
daß als Alkyloligoglycoside Verbindungen der Formel (II) eingesetzt werden, wobei Zₙ ein Oligoglycosylradikal bestehend aus 1,1 bis 1,45 Hexose- oder Pentoseeinheiten bzw. Mischungen davon bedeuten.

8. Tensidkombination nach Anspruch 1,
dadurch gekennzeichnet,
daß als elektrolytische Verdickungsmittel Natriumchlorid, Ammoniumchlorid, Natriumsulfat oder Magnesiumsulfat eingesetzt werden.

9. Tensidkombination nach Anspruch 1,
dadurch gekennzeichnet,
daß als Additive schaumstärkende, anionische Tenside verwendet werden.

10. Tensidkombination nach Anspruch 1,
dadurch gekennzeichnet,
daß das Verhältnis von a) : b) zwischen 5 : 1 und 1 : 10 beträgt.

11. Tensidkombination nach Anspruch 1,
dadurch gekennzeichnet,
daß das Verhältnis von a) : b) zwischen 2 : 1 und 1 : 5 beträgt.

## Claims

1. A surfactant combination for bath gels, shampoos and cleansing agents composed of
a) 3 - 30% by weight of carboxymethylated alkenol ethoxylate on its own
or a combination of carboxymethylated alkenol ethoxylate with carboxymethylated alkanol ethoxylate
or carboxymethylated alkylphenol ethoxylate having from 7 to 18 C atoms in the straight-chain or branched alkyl group on its own
or a combination of carboxymethylated alkylphenol ethoxylate having from 7 to 18 C atoms in the straight-chain or branched alkyl group with carboxymethylated alkenol ethoxylate,
b) 3 - 30% by weight of alkyl oligoglycoside,
c) 0.05 - 5% by weight of electrolytic thickener,
d) additives if desired and
water to 100% by weight.

2. A surfactant combination according to claim 1, characterised in that the carboxymethylated ethoxylates employed are compounds of the formula (I)
R-(OC₃H₆)ₘ(OC₂H)ₙOCH₂COOM (I)
in which R denotes a linear or branched, unsaturated aliphatic radical having from 10 to 20 carbon atoms, m denotes from zero to 10, n denotes from 1 to 15, and M denotes an alkali metal, alkaline earth metal, ammonium or alkylammonium ion, the degree of carboxymethylation being from 20 to 100% by weight.

3. A surfactant combination according to claim 2, characterised in that in the formula (I) R denotes a mono- or polyunsaturated hydrocarbon radical.

4. A surfactant combination according to claim 1, characterised in that the carboxymethylated ethoxylates employed are compounds of the formula (I)
R-(OC₃H₆)ₘ(OC₂H₄)ₙOCH₂COOM (I)
in which R denotes a linear or branched, saturated aliphatic radical having from 10 to 20 carbon atoms or an alkylaromatic radical having from 7 to 18 carbon atoms in the straight-chain or branched, saturated or unsaturated alkyl group and a linear or branched unsaturated aliphatic radical having from 10 to 20 carbon atoms, m is from zero to 10, n is from 1 to 15, and M is an alkali metal, alkaline earth metal, ammonium or alkylammonium ion, the degree of carboxymethylation being from 20 to 100% by weight.

5. A surfactant combination according to claim 1, characterised in that the alkyl oligoglycosides employed are compounds of the formula (II)
R'-O-Zₙ (II)
in which R' denotes a saturated or unsaturated, branched or unbranched alkyl radical having from 8 to 16 carbon atoms, and Zₙ denotes an oligoglycosyl radical composed of from 1 to 1.45 hexose or pentose units or mixtures thereof.

6. A surfactant combination according to claim 5, characterised in that the alkyl oligoglycosides employed are compounds of the formula (II) in which R' denotes a saturated or unsaturated, branched or unbranched alkyl radical having from 9 to 14 carbon atoms.

7. A surfactant combination according to claim 5, characterised in that the alkyl oligoglycosides employed are compounds of the formula (II) in which Zₙ denotes an oligoglycosyl radical composed of from 1.1 to 1.45 hexose or pentose units or mixtures thereof.

8. A surfactant combination according to claim 1, characterised in that the electrolytic thickener employed is sodium chloride, ammonium chloride, sodium sulphate or magnesium sulphate.

9. A surfactant combination according to claim 1, characterised in that the additives used are foam-strengthening, anionic surfactants.

10. A surfactant combination according to claim 1, characterised in that the ratio a) : b) is from 5 : 1 to 1 : 10.

11. A surfactant combination according to claim 1, characterised in that the ratio a) : b) is from 2 : 1 to 1 : 5.

## Revendications

1. Combinaison surfactive pour gels de bain, shampooings et détergents, constituée par
a) 3 à 30 % en poids d'un oxéthylat d'alcénol carboxyméthylé seul ou d'une combinaison
d'oxéthyl at carboxyméthylé d'alcénol avec un oxéthylat carboxyméthylé d'alcanol,
ou d'oxéthylat carboxyméthylé d'alkyl-phénol comportant de 7 à 18 atomes de carbone dans le groupe alkyle linéaire ou ramifié seul, ou d'une combinaison d'oxéthylat carboxyméthylé d'alkyl-phénol comportant de 7 à 18 atomes de carbone dans le groupe alkyle linéaire ou ramifié, avec un oxéthylat carboxyméthylé d'alcénol,
b) 3 à 30 % en poids d'un oligo-glycoside alkylé,
c) 0,05 à 5 % en poids d'un épaississant électrolytique,
d) éventuellement des additifs et
de l'eau jusqu'à 100 % en poids.

2. Combinaison surfactive selon la revendication 1,
caractérisée par le fait que l'on utilise, comme oxéthylats carboxyméthylés, des composés de la formule (I)
R-(OC₃H₆)ₘ(OC₂H₄)ₙOCH₂COOM (I),
dans laquelle R représente un radical aliphatique non-saturé, linéaire ou ramifié, comportant de 10 à 20 atomes de carbone, m a une valeur de zéro à 10, n a une valeur de 1 à 15, M représente un ion de métal alcalin, un ion de métal alcalino-terreux, un ion ammonium ou un ion alkyl-ammonium, le degré de carboxyméthylation se situant entre 20 et 100 % en poids.

3. Combinaison surfactive selon la revendication 2,
caractérisée par le fait que dans la formule (I), R représente un radical hydrocarboné à insaturation simple ou multiple.

4. Combinaison surfactive selon la revendication 1,
caractérisée par le fait que l'on utilise, comme oxéthylats carboxyméthylés, des composés de la formule (I)
R-(OC₃H₆)ₘ(OC₂H₄)ₙOCH₂COOM (I),
dans laquelle R représente un radical aliphatique saturé, linéaire ou ramifié, comportant de 10 à 20 atomes de carbone, ou bien un radical alkyl-aromatique comportant de 7 à 18 atomes de carbone dans le groupe alkyle à chaîne droite ou ramifié, saturé ou non-saturé, et un radical aliphatique non-saturé, linéaire ou ramifié, comportant de 10 à 20 atomes de carbone, m a une valeur de zéro à 10, n une valeur de 1 à 15, M représente un ion de métal alcalin, un ion de métal alcalino-terreux,un ion ammonium ou un ion alkyl-ammonium, le degré de carboxyméthylation se situant entre 20 et 100 % en poids.

5. Combinaison surfactive selon la revendication 1,
caractérisée par le fait que l'on utilise, comme alkyloligo-glycosides, des composés de la formule (II)
R'-O-Zₙ (II),
R' représentant un radical alkyle saturé ou non-saturé, ramifié ou non-ramifié, comportant de 8 à 16 atomes de carbone, Zₙ représentant un radical oligo-glycosyle constitué par 1 à 1,45 unités hexose ou pentose, ou des mélanges de ces composés.

6. Combinaison surfactive selon la revendication 5,
caractérisée par le fait que l'on utilise, comme alkyloligo-glycosides, des composés de la formule (II), R' représentant un radical alkyle saturé ou non-saturé, ramifié ou non-ramifié, comportant de 9 a 14 atomes de carbone.

7. Combinaison surfactive selon la revendication 5,
caractérisée par le fait que l'on utilise, comme alkyloligo-glycosides, des composés de la formule (II), Zₙ représentant un radical oligo-glycosyle constitué par 1,1 à 1,45 unités hexose ou pentose, ou des mélanges de ces composés.

8. Combinaison surfactive selon la revendication 1,
caractérisée par le fait que l'on utilise, comme épaississant électrolytique, du chlorure de sodium, du chlorure d'ammonium, du sulfate de sodium ou du sulfate de magnésium.

9. Combinaison surfactive selon la revendication 1,
caractérisée par le fait que l'on utilise, comme additifs, des tensio-actifs anioniques renforçant la mousse.

10. Combinaison surfactive selon la revendication 1,
caractérisée par le fait que la proportion de a) à b) a une valeur comprise entre 5 : 1 et 1 : 10.

11. Combinaison surfactive selon la revendication 1,
caractérisée par le fait que la proportion de a) : b) est comprise entre 2 : 1 et 1 : 5.
